# FASCICULE DE BREVET EUROPEEN

(11) **EP 3 016 729 B1**
(45) Date de publication et mention de la délivrance du brevet: **25.03.2020**
(21) Numéro de dépôt: 14750552.3
(22) Date de dépôt: 04.07.2014
(51) Int. Cl.: B01D 15/38, A61K 39/395, B01J 20/289, B01J 20/32, C07K 16/06, C07K 1/22

(54) **MATRICE DE CHROMATOGRAPHIE D'AFFINITÉ**
AFFINITÄTSCHROMATOGRAFIEMATRIX
AFFINITY CHROMATOGRAPHY MATRIX

(30) Priorité: 05.07.2013 FR 1356635; 05.07.2013 FR 1356636
(43) Date de publication de la demande: 11.05.2016
(73) Titulaire: Laboratoire Français du Fractionnement et des Biotechnologies Société Anonyme, 91940 Les Ulis (FR)
(72) Inventeur: PAOLANTONACCI, Philippe, F-91190 Gif Sur Yvette (FR); CHTOUROU, Abdessatar, F-78990 Elancourt (FR)
(74) Mandataire: Cabinet Becker et Associés
(86) Numéro de dépôt international: PCT/FR2014/051734
(87) Numéro de publication internationale: WO 2015/001277

(56) Documents cités:
- EP-A1- 0 320 472
- EP-A1- 2 556 848
- WO-A1-2013/066251
- AU-A1- 2013 200 440
- US-A1- 2004 242 857
- BOEDEN H F ET AL: "Bead cellulose derivatives as supports for immobilization and chromatographic purification of proteins", JOURNAL OF CHROMATOGRAPHY A, ELSEVIER, AMSTERDAM, NL, vol. 552, 9 August 1991 (1991-08-09), pages 389-414, XP026514558, ISSN: 0021-9673, DOI: 10.1016/S0021-9673(01)95956-4 [retrieved on 1991-08-09]

## Description

La présente invention concerne une matrice pour la mise en œuvre d'une chromatographie d'affinité, destinée à retenir, et éliminer, des anticorps dirigés contre les antigènes du groupe sanguin A et/ou du groupe sanguin B, d'un produit sanguin.

### Arrière-plan technologique :

De nombreuses pathologies sont actuellement traitées par des compositions d'immunoglobulines (Ig). On peut citer par exemple les déficits immunitaires primitifs avec défaut de production d'anticorps, la maladie de Kawasaki, le purpura thrombopénique immunologique de l'enfant et de l'adulte, les déficits immunitaires secondaires avec défaut de production d'anticorps, en particulier la leucémie lymphoïde chronique ou myélome associés à des infections à répétition, l'infection de l'enfant par le VIH associé à des infections bactériennes, les neuropathies motrices multifocales, le syndrome de Guillain-Barré, les infections aiguës sévères ou chroniques à Parvovirus B19, l'immunodéficience acquise ou constitutionnelle, la dermatomyosite cortico-résistante, la myasthénie aiguë, la polyradiculonévrite chronique idiopathique, le purpura thrombopénique immunologique, par exemple associé à l'infection par le VIH, le syndrome de l'homme raide (Stiffman syndrome), la neutropénie auto-immune, l'érythroblastopénie auto-immune résistante, le syndrome d'anti-coagulation acquise par auto-anticorps, la polyarthrite rhumatoïde, les uvéites, etc.

L'utilisation grandissante de fractions de plasma humain enrichies en Ig en thérapie nécessite la production à grande échelle, à partir par exemple de plasmas humains, de concentrés d'Ig hautement purifiées, injectables par voie intraveineuse (IgIV), ou sous-cutanée (Igsc).

Or cette production fait face à de nombreuses contraintes, notamment en termed'innocuité des concentrés d'Ig obtenus.

Selon la Pharmacopée Européenne, afin de réduire les risques d'hémolyse, ces concentrés doivent présenter une teneur réduite en anticorps dirigés contre les antigènes du groupe sanguin A et/ou du groupe sanguin B (désignés dans la description, de manière abrégée, « anticorps anti- groupe sanguin A » et « anti- groupe sanguin B », ou encore « anticorps anti-A » et « anticorps anti-B », ou encore « isoagglutinines anti-A » et « isoagglutinines anti-B »).

Etant donné que les besoins en Ig sont en constante augmentation, il est nécessaire de disposer de pools de plus en plus importants de donneurs, qui seront statistiquement d'autant plus riches en groupe sanguin O. Il est donc devenu essentiel de disposer de procédés permettant d'éliminer les anticorps anti-A et anti- B des produits sanguins, au cours de la production industrielle des concentrés d'Ig à partir des pools de plasmas sanguins.

La demande WO2007/077365 décrit un procédé de préparation de concentrés d'Ig, comprenant une étape de chromatographie d'immunoaffinité visant à appauvrir ceux-ci en anticorps anti-A et anti-B.

La demande WO2013066251 concerne un procédé pour réduire ou éliminer un composant d'un produit sanguin en utilisant une circulation extracorporelle. Ce procédé peut comprendre une étape dans laquelle le produit sanguin est traité avec un absorbant sur lequel sont immobilisés des ligands d'intérêt, notamment des peptides ou des trisaccharides du groupe sanguin A ou B. WO2013066251 décrit plusieurs espaceurs utilisables pour immobiliser des ligands sur la matrice de l'absorbant.

La demande AU2013200440 décrit un procédé de préparation d'un concentré d'immunoglobulines dépourvu d'anticorps anti-A et anti-B, à usage thérapeutique, comprenant une étape d'élimination des anticorps anti-A et anti-B par la mise en œuvre d'une étape de chromatographie d'affinité à l'aide de matrices sur lesquelles sont greffés des ligands oligosaccharides présentant une similarité avec les groupes sanguins A et B.

### Résumé de l'invention :

La Demanderesse a maintenant mis au point un support, particulièrement utile pour retenir, et éliminer, des anticorps anti-A et/ou anti-B d'un produit sanguin, par chromatographie d'affinité à l'échelle industrielle.

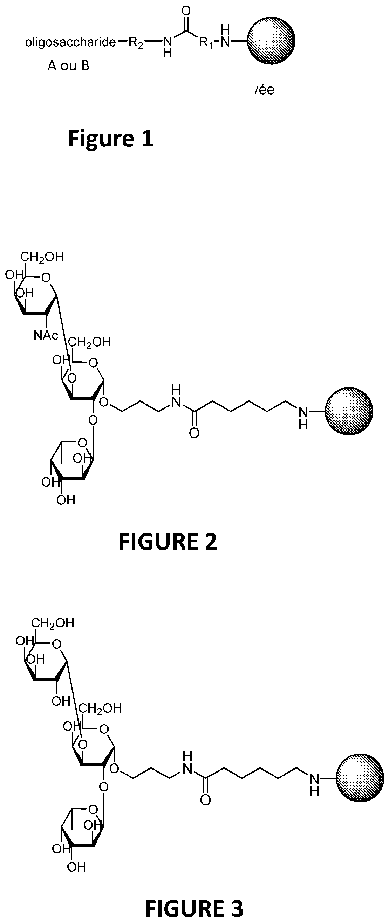

L'invention fournit ainsi une matrice de chromatographie d'affinité, sous forme de gel, comprenant des particules de polymère sur lesquelles au moins un oligosaccharide correspondant à un épitope de groupe sanguin A et/ou de groupe B est greffé, ledit oligosaccharide étant greffé auxdites particules via un espaceur, caractérisée en ce que la la densité d'oligosaccharides est d'environ 0,3 à 0,4 mg/ml de matrice et ledit espaceur consiste en un espaceur de formule -NH-C₅H₁₀-CO-NH-C₃H₆-, ledit espaceur étant lié par sa fonction amine à la particule. Un schéma de ces particules greffées est présenté à la figure 1.

Dans un mode de réalisation particulier, la matrice comprend (i) des particules de polymère sur lesquelles au moins un oligosaccharide correspondant à un épitope de groupe sanguin A est greffé, et/ou (ii) des particules de polymère sur lesquelles au moins un oligosaccharide correspondant à un épitope de groupe sanguin B est greffé.

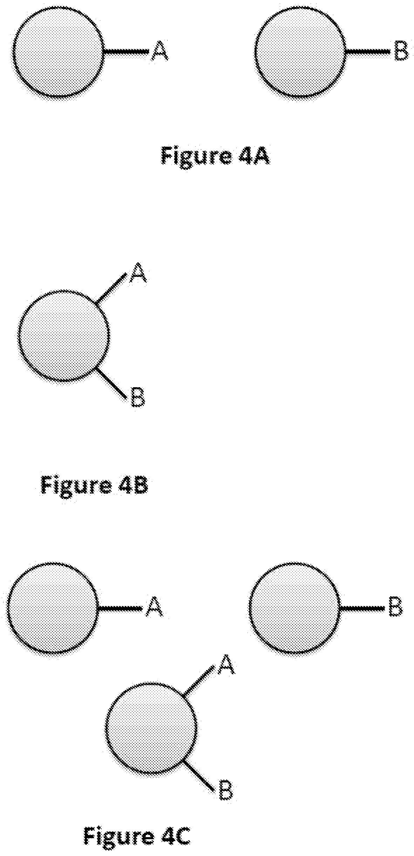

Dans un mode de réalisation préféré, la matrice comprend (i) des particules de polymère sur lesquelles au moins un oligosaccharide correspondant à un épitope de groupe sanguin A est greffé, et (ii) des particules de polymère sur lesquelles au moins un oligosaccharide correspondant à un épitope de groupe sanguin B est greffé. Ce mode de réalisation est illustré à la **Figure 4A****.**

Dans un autre mode de réalisation, la matrice comprend des particules de polymère sur lesquelles à la fois au moins un oligosaccharide correspondant à un épitope de groupe sanguin A, et au moins un oligosaccharide correspondant à un épitope de groupe sanguin B, sont greffés. Ce mode de réalisation est illustré à la **Figure 4B****.**

Dans un mode de réalisation particulier, la matrice comprend un mélange de (i) particules de polymère sur lesquelles au moins un oligosaccharide correspondant à un épitope de groupe sanguin A est greffé, (ii) particules de polymère sur lesquelles au moins un oligosaccharide correspondant à un épitope de groupe sanguin B est greffé, et (iii) particules de polymère sur lesquelles à la fois au moins un oligosaccharide correspondant à un épitope de groupe sanguin A, et au moins un oligosaccharide correspondant à un épitope de groupe sanguin B, sont greffés. Ce mode de réalisation est illustré à la **Figure 4C****.**

Dans tous les modes de réalisation, l'espaceur qui lie le ligand correspondant à un épitope de groupe sanguin A à la particule, et l'espaceur qui lie le ligand correspondant à un épitope de groupe sanguin B à la particule peuvent être identiques ou différents, de préférence identiques, mais toujours de formule (I).

L'invention vise encore l'utilisation de cette matrice, ou support, dans une chromatographie d'affinité liant des anticorps anti-A et/ou anti-B.

L'invention vise aussi l'utilisation de cette matrice, ou support, pour la production à l'échelle industrielle d'immunoglobulines G (IgG) à usage thérapeutique.

L'invention fournit également un procédé de préparation d'un concentré d'immunoglobulines (Ig) à usage thérapeutique, comprenant l'obtention d'une composition d'Ig à partir de plasma sanguin, par fractionnement éthanolique et/ou fractionnement caprylique et/ou séparation chromatographique, et l'élimination des anticorps anti- A et/ou anti-B éventuellement présents dans la composition, au moyen d'une chromatographie d'affinité mettant en œuvre la matrice telle que définie ici.

Un avantage de cette matrice est sa grande sélectivité et spécificité vis-à-vis des anticorps anti-A et anti-B, et sa grande capacité de liaison à ceux-ci. Par conséquent, il devient possible de réduire le temps de traitement des produits à l'échelle industrielle, en faisant passer une plus grande quantité de produit par volume de colonne.

### Légendes des Figures :

La **Figure 1** représente un schéma simplifié d'une particule de polymère sur laquelle un oligosaccharide correspondant à un épitope de groupe sanguin A ou B est greffé. Selon l'invention, R₁ et R₂ sont tels que l'espaceur est de formule: -NH-C₅H₁₀-CO-NH-C₃H₆-.
La **Figure 2** représente un schéma simplifié d'un mode de réalisation préféré de l'invention, représentant une particule de polymère sur laquelle un trisaccharide correspondant à un épitope de groupe sanguin A est greffé.
La **Figure 3** représente un schéma simplifié d'un mode de réalisation préféré de l'invention, représentant une particule de polymère sur laquelle un trisaccharide correspondant à un épitope de groupe sanguin B est greffé.
La **Figure 4A** représente un schéma simplifié d'un mode de réalisation particulier de l'invention, d'une matrice comprenant un mélange de particules portant un oligosaccharide correspondant à un épitope de groupe sanguin A, et de particules portant un oligosaccharide correspondant à un épitope de groupe sanguin B.
La **Figure 4B** représente un schéma simplifié d'un mode de réalisation particulier de l'invention, d'une matrice comprenant des particules portant à la fois un oligosaccharide correspondant à un épitope de groupe sanguin A, et un oligosaccharide correspondant à un épitope de groupe sanguin B.
La **Figure 4C** représente un schéma simplifié d'un mode de réalisation particulier de l'invention, d'une matrice comprenant un mélange de particules portant un oligosaccharide correspondant à un épitope de groupe sanguin A, de particules portant un oligosaccharide correspondant à un épitope de groupe sanguin B, et de particules portant à la fois un oligosaccharide correspondant à un épitope de groupe sanguin A, et un oligosaccharide correspondant à un épitope de groupe sanguin B.

### Description détaillée de l'invention :

### Le support :

La matrice de l'invention comprend un support à base de particules de polymère, et est sous forme d'un gel.

Ces particules de polymère sont de préférence de forme sphérique ou oblongue, il peut s'agir notamment de billes. Ces particules ont généralement une taille moyenne d'environ 0.1 µm à environ 1000 µm, de préférence d'environ 20 à environ 500µm, de préférence encore d'environ 50 à environ 200µm, de préférence encore d'environ 70µm à environ 120µm de diamètre.

De préférence ces particules sont poreuses.

Le polymère peut être naturel ou non-naturel, organique ou inorganique, réticulé ou non réticulé.

Le polymère est de préférence un polymère organique, de préférence réticulé.

Dans un mode de réalisation préféré, le polymère est de la cellulose, et les particules sont de préférence des billes de cellulose poreuses.

De préférence encore, il s'agit de cellulose réticulée.

D'autres types de polymères possibles incluent agarose, dextran, polyacrylates, polystyrène, polyacrylamide, polyméthacrylamide, des copolymères de styrène et divinylbenzène, ou des mélanges de ces polymères.

Les particules peuvent fournir un milieu de chromatographie que l'on peut utiliser pour remplir une colonne par exemple.

### Les ligands :

Les ligands portés par le support selon l'invention sont des oligosaccharides représentant les antigènes des groupes sanguins A ou B, qui sont naturellement des oses.

Plus précisément l'oligosaccharide correspondant à un épitope de groupe sanguin A et/ou l'oligosaccharide correspondant à un épitope de groupe sanguin B porté(s) par la matrice selon l'invention sont typiquement des trisaccharides. Le terme « oligosaccharides correspondants à un épitope de groupe sanguin» se réfère à des motifs identiques ou similaires, d'un point de vue antigénique, aux déterminants antigéniques reconnus par les anticorps anti-A et anti-B, respectivement. Les ligands portés par le support selon l'invention sont donc spécifiques des anticorps anti-A ou anti-B.

De préférence l'oligosaccharide correspondant à un épitope de groupe sanguin A, utilisé comme ligand dans l'invention, est un trisaccharide N-acétylgalactosamine (GalNAc)-Galactose(Gal)-Fucose, plus précisément N-acétylGalα1-3(Fuc α1-2)Gal, l'espaceur est lié par l'atome d'oxygène lié de préférence au carbone en position 1 du galactose :

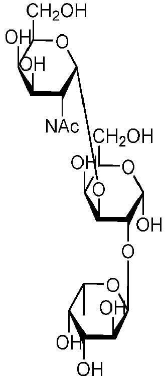

L'oligosaccharide correspondant à un épitope de groupe sanguin B, utilisé comme ligand dans l'invention, est de préférence un oligosaccharide Galactose-Galactose-Fucose, plus précisément Gal α1-3((Fuc α1-2)Gal, l'espaceur est lié par l'atome d'oxygène lié de préférence au carbone en position 1 du galactose :

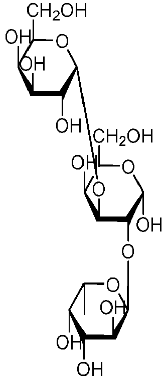

### L'espaceur :

Le ligand est lié de manière covalente à l'espaceur, qui lie le ligand aux particules.

L'espaceur permet de réduire l'encombrement stérique et d'augmenter l'accessibilité du ligand vis-à-vis des anticorps anti-A et anti-B à lier.

L'espaceur sert à immobiliser, sur une particule, soit un oligosaccharide correspondant à un épitope de groupe sanguin A, qui est de préférence un trisaccharide N-acétylGalα1-3(Fuc α,1-2)Gal tel que décrit ci-dessus, soit un oligosaccharide correspondant à un épitope de groupe sanguin B, qui est de préférence un trisaccharide Gal α1-3((Fuc α1-2)Gal tel que décrit ci-dessus.

Une particule peut porter plusieurs espaceurs.

La liaison entre le ligand et l'espaceur peut être, par exemple, une liaison amide. L'espaceur comprend typiquement au moins un atome de C, O, N, ou S.

Il est décrit des espaceurs de type -(CH2)mX(CH2)n- ou -(CH2)mX1(CH2)nX2(CH2)p-, où X, X1, et X2 sont chacun indépendamment l'un de l'autre choisi parmi O, S, NH, et une liaison covalente; et m, n, et p sont chacun indépendamment 0, 1, 2, 3, 4, 5, ou 6. 1, 2, ou 3 des atomes d'hydrogène ci-dessus peuvent être remplacés par un nombre équivalent de groupes OH et/ou méthyle.

Il est également décrit un espaceur comprenant une structure choisie parmi 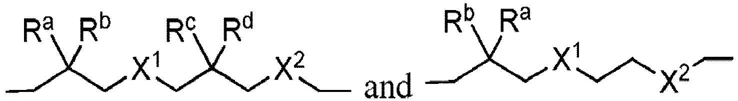 où chacun de X1 et X2 est choisie indépendamment parmi O, S, et NH; et chacun de Ra, Rb, Rc, and Rd est choisie indépendamment parmi H, OH, et méthyle.

Il est aussi décrit un espaceur comprenant une structure choisie parmi

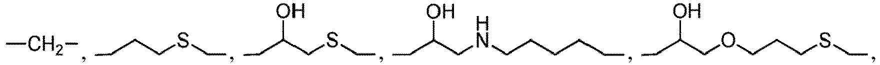

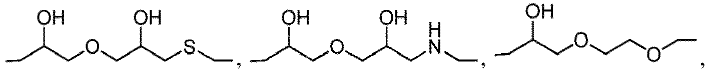

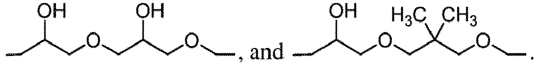

Il est également décrit un espaceur qui présente la formule (I) **-NH-**R1-CO-NH-R2-, dans laquelle R1 est un groupe alkyle en C4-C6, R2 est un groupe alkyle en C3-C8, et ledit espaceur est lié par sa fonction amine à la particule (en gras ci-dessus). Un schéma de cette matrice est présenté à la **Figure 1****.**

R1 est un groupe alkyle en C4-C6, linéaire ou ramifié, de préférence linéaire. Selon l'invention, R1 est un groupe alkyle en C5.

R2 est un groupe alkyle en C3-C8, linéaire ou ramifié, de préférence linéaire. Selon l'invention, R2 est un groupe alkyle en C3.

Selon l'invention, les ligands (qui sont de préférence des trisaccharides tels que décrits plus haut) sont greffés aux particules par un espaceur qui consiste en un espaceur de formule : (particule)-NH-C₅H₁₀-CO-NH-C₃H₆-(ligand).

Dans un mode de réalisation préféré, sont mélangées billes de cellulose réticulée sur lesquelles sont greffés des ligands qui sont des trisaccharides correspondant à un épitope du groupe sanguin A (N-acétylgalactosamine (GalNAc)-Galactose(Gal)-Fucose), comme représentées à la **Figure 2****,** et billes de cellulose réticulée sur lesquelles sont greffés des ligands qui sont des trisaccharides correspondant à un épitope du groupe sanguin B (Galactose-Galactose-Fucose), comme représentées à la **Figure 3****.** Les trisaccharides sont greffés aux billes par un espaceur de formule : (bille)-NH-C₅H₁₀-CO-NH-C₃H₆-(ligand)

### La préparation de la matrice :

Les ligands sont immobilisés chimiquement par des liaisons covalentes entre les particules et l'espaceur, et entre l'espaceur et le ligand.
Cette immobilisation peut être réalisée de manière classique par l'homme du métier. Dans un mode de réalisation préféré, la particule porte un bras -NH-R1-COOH dans laquelle R₁ est C₅H₁₀ de telle sorte qu'il s'agisse d'acide ε-aminocaproïque (où R1 est un groupe pentyle).
De manière classique, la particule peut être activée en utilisant des réactifs bifonctionnels tels que tels que l'épichlorhydrine, répibromhydrine, dibromo-et dichloropropanol, dibromobutane, l'éthylène glycol diglycidyléther, le butanediol diglycidylether, divinylsulfone, allylglycidyléther, et le bromure d'allyle. Le réactif bifonctionnel est capable de réagir à la fois avec les particules et le bras -NH-R1-COOH. Les composés hétérofonctionnels allyle, tels que le bromure d'allyle, sont des réactifs bifonctionnels préférés et permettent d'obtenir une matrice activée.
Pour certains supports solides, tels que la cellulose, les composites contenant un hydrogel ou d'autres matériaux présentant des groupes hydroxyles, il est avantageux de déprotoner les groupes hydroxyles avec une source d'hydroxyde, par exemple, avant la réaction avec un réactif bifonctionnel.
Les ligands représentant les antigènes des groupes sanguins A et/ou B sont ensuite immobilisés sur la particule activée portant le bras -NH-R1-COOH via un groupe lieur - NH-R2-, où R2 est -C₃H₆-. Pour cela, la fonction COOH du bras -NH-R1-COOH portée par la particule est mis à réagir avec la fonction NH₂ du ligand NH₂-R2-oligosacccharide, par la mise en œuvre d'un agent de condensation de type N-éthoxycarbonyl-2-éthoxy-1,2-dihydroquinoline (EEDQ).

L'homme du métier peut greffer soit un ligand représentant un antigène de groupe A soit un ligand représentant un antigène de groupe B, soit encore les deux, sur la même particule. De préférence, l'espaceur utilisé est identique. Ceci est particulièrement avantageux pour assurer un greffage équivalent de ligands représentant un antigène de groupe A par rapport aux ligands représentant un antigène de groupe B. L'homme du métier sait par ailleurs se placer dans des conditions adaptées, en fonction de la réactivité des ligands vis-à-vis des particules, pour obtenir des particules portant une proportion déterminée de ligands représentant un antigène de groupe A par rapport aux ligands représentant un antigène de groupe B.

La matrice sous forme de gel est préparée par addition classique d'un tampon sur les particules de polymère portant les ligands, comme cela est connu de l'homme du métier, de manière à obtenir une matrice sous forme de gel, adaptée pour une chromatographie d'affinité.

Il est décrit des matrices dans lesquelles la densité de ligands, c'est-à-dire la quantité de ligands (à savoir d'oligosaccharides correspondant à un épitope de groupe sanguin A ou B) greffés, par volume de matrice, est comprise entre environ 0,2 et environ 0,7 mg/ml de matrice.
La matrice de chromatographie d'affinité selon l'invention a une densité d'oligosaccharides comprise environ 0,3 et environ 0,4 mg/ml de matrice. De préférence la densité d'oligosaccharides est d'environ 0,3mg/ml de matrice.
Selon l'invention, cette densité permet de diminuer de manière drastique le temps de purification par chromatographie d'affinité, par comparaison avec une densité de 1mg/ml (par exemple sur gel GLYCOSORB ABO® de chez Glycorex Transplantation AB (Suède), mentionné dans la demande WO2007/077365). Le volume de matrice peut être augmenté et le débit à travers la colonne de chromatographie peut être augmenté. Ainsi, avec une densité de ligands de 0,3mg/ml de matrice (comparée à une densité de 1mg/ml), la durée du procédé a été divisée par 2, ce qui, à l'échelle industrielle, représente un gain remarquable. De manière avantageuse, la diminution de la densité de ligands permet également de diminuer le coût de la matrice, et rend ainsi l'étape d'affinité sur la matrice selon l'invention moins coûteuse, contribuant à baisser le prix de revient de la composition d'immunoglobulines finale. Avantageusement, la diminution de la densité de ligands s'accompagne d'une conservation de la capacité d'élimination des anticorps anti-A et/ou anti-B.

Dans un mode de réalisation préféré, les particules de polymère sur lesquelles au moins un oligosaccharide correspondant à un épitope de groupe sanguin A est greffé, et les particules de polymère sur lesquelles au moins un oligosaccharide correspondant à un épitope de groupe sanguin B est greffé, peuvent ensuite être mélangées, par exemple dans une proportion de 25/75 à 75/25 (v/v), de préférence environ 50/50 (v/v).

Les particules de polymère sur lesquelles au moins un oligosaccharide correspondant à un épitope de groupe sanguin A est greffé, et les particules de polymère sur lesquelles au moins un oligosaccharide correspondant à un épitope de groupe sanguin B est greffé, peuvent également, le cas échéant, être mélangées à des particules de polymère portant à la fois au moins un oligosaccharide correspondant à un épitope de groupe sanguin A, et au moins un oligosaccharide correspondant à un épitope de groupe sanguin B.

### Chromatographie d'affinité :

La matrice telle que définie ici est utile dans une chromatographie d'affinité liant des anticorps anti- A et/ou anti- B.

Pour cela, la matrice peut être introduite dans une colonne de chromatographie. A l'échelle industrielle, la colonne peut contenir de 1 à 150 litres, voire 250 à 500 L, si nécessaire. Dans le cas d'une mise en œuvre à l'échelle pilote, on peut utiliser des colonnes de 1 à 50 cm de hauteur, le diamètre est alors adapté à la hauteur de colonne utilisée. Le volume de matrice utilisé dans la colonne est ajusté en fonction du taux résiduel en anticorps anti-A et/ou anti-B souhaité par rapport au taux d'anticorps anti-A et/ou anti-B initial de la solution. Le volume de matrice peut également être ajusté pour répondre aux contraintes d'un procédé industriel, notamment être adapté aux volumes de produit à traiter.

Les anticorps anti-A et/ou anti-B présents dans la préparation liquide d'immunoglobulines (ou le dérivé de produits sanguins) se fixent à la matrice, et le produit non adsorbé est récupéré, appauvri en anticorps anti-A et/ou anti-B. La préparation liquide d'immunoglobulines ou le dérivé de produits sanguins est versé à travers la matrice à une vitesse et dans des conditions qui permettent la liaison des anticorps avec les ligands portés par la matrice.

Les matrices peuvent être ré-utilisées de manière répétée, par exemple jusqu'à 100 fois environ, sans dégradation. Leur régénération peut être réalisée par des procédures connues de l'homme du métier, par exemple par traitement par de la soude (NaOH), par exemple à 1 M. La réutilisation après régénération répond aux exigences de sécurité d'un procédé industriel de fabrication de médicaments, notamment aux besoins de décontamination biologique et à l'élimination des traces de produit pouvant induire une contamination lot à lot. La réutilisation de la matrice d'affinité permet aussi avantageusement de baisser le prix de revient industriel lors de la fabrication d'immunoglobulines.

La matrice d'affinité selon l'invention est donc parfaitement adaptée à une utilisation industrielle pour la fabrication de médicaments tels que les immunoglobulines, en permettant une élimination robuste et suffisante des anticorps anti-A et/ou anti-B initialement présents dans la solution, sans augmenter de manière drastique le prix de revient industriel du produit d'immunoglobulines final.

### Purification des produits d'immunoglobulines :

Une chromatographie d'affinité utilisant la matrice de l'invention est particulièrement avantageuse pour purifier des préparations ou compositions d'immunoglobulines, en éliminant les anticorps anti-A et/ou anti-B indésirables éventuellement présents dans ces préparations ou compositions, et ainsi préparer des concentrés d'immunoglobulines à usage thérapeutique.
Par « élimination », on entend une réduction substantielle de la quantité d'anticorps anti-A et/ou anti-B présents, de préférence d'au moins 25%, de préférence encore d'au moins 50%, encore plus préférentiellement d'au moins 80 ou 90%. Le concentré d'Ig de l'invention obtenu après chromatographie d'affinité présente des teneurs respectives en anticorps anti-A et anti-B conformes (à la dilution 1/64) à un résultat négatif au test de Coombs direct mis en œuvre avec une concentration initiale d'IgG à 30g/l. De préférence, le concentré d'Ig de l'invention obtenu après chromatographie d'affinité comprend une teneur en anticorps anti-A non supérieure à 23 ng/mg d'IgG, et une teneur en anticorps anti-B non supérieure à 20 ng/mg d'IgG. Des méthodes de dosage des anticorps anti-A et anti-B résiduels sont décrites notamment dans la demande WO2007/077365. De préférence, une méthode de dosage des anticorps anti-A et anti-B est effectuée par une cytométrie de flux, dont le principe repose sur l'utilisation d'hématies humaines de groupe A ou B, selon la détermination spécifique du titre des anticorps anti-A et anti-B voulue, mettant en œuvre la détection d'un signal de fluorescence proportionnel à la teneur en ces anticorps. La méthode de dosage par cytométrie de flux utilise généralement un échantillon standard, par exemple un contrôle positif d'immunoglobulines (tel qu'un titre 1:32 de EDQM, ref#07/306; Thorpe et al. 2009, Vox Sang. 97, 160-168), ou un concentré d'anticorps anti-D monoclonal ou tout autre produit de référence adapté.

Les produits d'immunoglobulines contiennent essentiellement des IgG. Typiquement, ces IgG sont des IgG polyclonales obtenues à partir du plasma sanguin ou d'une fraction de plasma sanguin déjà enrichie en Ig.
Les concentrés d'Ig à usage thérapeutique sont à des concentrations comprises entre 50 et 100 g/1. Ces concentrés sont destinés à un usage clinique et peuvent en particulier être injectés par voie intraveineuse. A cet effet, ils doivent être sécurisés et, le cas échéant, contenir des excipients, tels que des stabilisants, compatibles avec cet usage clinique. Les concentrés d'Ig à usage thérapeutique peuvent également être administrés par voie sous-cutanée. Dans ce cas, la concentration des produits est supérieure ou égale à 100g/l, avantageusement supérieure ou égale à 150 g/l.
Les concentrés d'Ig à usage thérapeutique peuvent aussi être administrés par voie intramusculaire.
Ces concentrés d'Ig peuvent être obtenus de la manière suivante:
a) préparation d'une composition d'Ig, par exemple par fractionnement éthanolique et/ou par fractionnement caprylique et/ou par séparation chromatographique,
b) chromatographie d'immunoaffinité par percolation de la composition d'Ig sur une matrice de l'invention,
et c) étape de sécurisation biologique, de préférence une nanofiltration de manière à éliminer virus et/ou particules contaminantes.

La composition d'Ig peut être obtenue par un fractionnement éthanolique développé à l'origine par Cohn et al (Cohn et al, 1946. J. Am. Chem. Soc. 68, 459 ; Oncley et al, 1949, J. Am. Chem. Soc. 71, 541), ou bien par une séparation chromatographique, telle que décrite par exemple dans EP 0 703 922 et WO 99/64462, ou encore par un fractionnement caprylique tel que décrit par Steinbuch et al 1969, Arch Biochem Biophys. 134(2):279-84). On préfère tout particulièrement les procédés mis au point par la Demanderesse dans les demandes de brevet WO 94/29334 et WO 02/092632, et tout particulièrement celui décrit dans WO 02/092632. Dans ce cas, on soumet un plasma sanguin, ou une fraction de plasma sanguin enrichie en IgG, à un fractionnement caprylique (prépurification par précipitation de contaminants non immunoglobulines), une chromatographie unique sur un support de résine échangeuse d'anions effectuée à pH alcalin, une élution sélective des IgG en une étape par un tampon approprié à pH compris entre 4 et 7. Un traitement d'inactivation virale peut être réalisé, de préférence effectué par solvant-détergent, comme décrit par Horowitz dans le brevet US 4 764 369.
La fraction d'IgG ainsi récoltée est déjà concentrée, mais peut ensuite subir des étapes de concentration supplémentaire par ultrafiltration, et de filtration stérilisante.
Ce concentré est ensuite soumis à une étape chromatographique d'immunoaffinité sur la matrice de l'invention. De préférence, le pH de la préparation d'immunoglobulines est ajusté à un pH d'environ 6 à environ 7, de préférence d'environ 6, avant la chromatographie.
La charge de la colonne est adaptée au taux résiduel recherché d'anticorps anti-A et/ou anti-B. La spécificité d'une telle matrice ne nécessite pas un conditionnement préalable de la fraction d'IgG, c'est-à-dire que toute fraction ou concentré d'IgG obtenue par les techniques de fractionnement de plasma peut convenir.
La percolation du concentré ne fait pas intervenir de mécanisme d'élution. Par conséquent, quelle que soit la manière dont est obtenu le concentré d'IgG, il est percolé à travers la colonne, éventuellement grâce à une pompe. Cette percolation permet la rétention des anticorps anti-A et anti-B. Le temps de contact entre les ligands et la préparation d'Ig est supérieur ou égal à une minute, avantageusement de l'ordre de 2 minutes.
La colonne peut être ensuite lavée par de l'eau pour récupérer les IgG encore présentes dans le volume mort de la colonne.
Apres percolation du concentré d'IgG, on obtient une fraction d'IgG appauvrie en anticorps anti-A et anti-B.
Le procédé peut comprendre, ensuite des étapes de concentration par ultrafiltration et de filtration stérilisante.

La colonne chromatographique et la matrice peuvent ensuite être lavées et éluées, pour désorber les anticorps anti-A et anti-B retenus.

L'exemple qui suit illustre la présente invention sans toutefois en limiter la portée.

### Exemple : Chromatographie d'affinité (échelle industrielle)

### Conditions de chromatographie :

Une préparation d'immunoglobulines, obtenue par fractionnement selon le procédé décrit dans la demande WO2002/092632, comprenant 10 ±2 g/l d'IgG, est soumise, à une échelle industrielle, à une étape de chromatographie d'affinité anti-A/ anti-B réalisée sur une colonne comprenant un mélange 50/50 (v/v) de billes de cellulose réticulée sur lesquelles sont greffés des trisaccharides correspondant à un épitope du groupe sanguin A (N-acétylgalactosamine (GalNAc)-Galactose(Gal)-Fucose), comme représentées à la **Figure 2** (gel désigné « Iso A HyperCel »), et de billes de cellulose réticulée sur lesquelles sont greffés des trisaccharides correspondant à un épitope du groupe sanguin B (Galactose-Galactose-Fucose), comme représentées à la **Figure 3** (gel désigné « Iso B HyperCel »).
Les trisaccharides sont greffés aux billes par un espaceur de formule : (bille)-NH-C₅H₁₀-CO-NH-C₃H₆-(ligand)
La densité de trisaccharides greffés est de 0,3mg par ml de gel de matrice.

Pour vérifier l'activité résiduelle anti-A et anti-B, la préparation d'immunoglobulines désignée Eluat (fraction obtenue après chromatographie échangeuse d'anions), dont le pH a été ajusté à 6 (± 0.05) a été passée sur une colonne de 1 ml (comprenant 0,5 ml de gel Iso A HyperCel + 0,5 ml de gel Iso B HyperCel)
Format de la colonne : D = 0,5 cm x 5,1 cm (volume de la colonne (VC) = 1 ml).
Temps de contact : 2 min
Charge : 6g de préparations Eluat / mg de ligands A+B, soit 180 ml de produit par colonne.
Avant l'essai, le gel est lavé à l'eau (10 VC minimum).

Les conditions des différentes étapes du procédé sont résumées dans le tableau ci-dessous :

**Tableau des étapes :**

| **Etape** | **Solution** | **Volume** |
|---|---|---|
| Sanitisation | NaOH 1M (30min) | 6VC |
| | Phosphate 1M pH 8,0 | 4VC |
| Equilibrage | Eau PPI (Retour à pH neutre 5 à 7) | 10VC |
| Fixation | Eluat | ± 180ml |
| Collecte non fractionnée de la FNA | | |
| Lavage | NaCl 150mM + Phosphate 15mM pH 6,2 | 10VC |
| Elution acide | Glycine 0,1M pH 3 | 6VC |
| Elution basique | Glycine 0,1M pH 11 | 6VC |

| | | |
|---|---|---|
| VC: volume colonne; FNA : Fraction non adsorbée | | |

Une seule Fraction non adsorbée (FNA) a été recueillie.

### Tests de dosage :

Le rendement a été déterminé par dosage des IgG dans la fraction non adsorbée, après lavage et percolation. L'activité résiduelle anti-A et anti-B, correspondant au pourcentage d'isoagglutinines anti-A et anti-B présentes dans la préparation finale par rapport à leur concentration avant l'étape de chromatographie d'affinité, a été mesurée par cytométrie en flux (technique plus sensible et précise que celle des dilutions demandée par la Pharmacopée Européenne), selon la technique décrite ci-dessous.

Des globules rouges de groupes A- et B- ont été recueillis sur EDTA et lavés deux fois dans une solution de NaCl 0.9% (centrifugation à 1730 g pendant 5 min entre les deux lavages). 2.10⁶ globules rouges ont été distribués sur une plaque de microtitration et on a ajouté 50 µL de contrôle positif standard (1:32 EDQM, ref#07/306; Thorpe et al. 2009) ou les échantillons dilués à une concentration de travail (PBS pH 7.4, 1% BSA). Les plaques ont été incubées pendant 2h à 37°C sous agitation. Après lavage, un anticorps F(ab')² de chèvre anti-IgG humaines (Fc spécifique) (Beckman Coulter) marqué à la phycoerythrine (PE) a été utilisé dilué à 1/20 dans du PBS-BSA. Les plaques ont été incubées 30 min à température ambiante et à l'abri de la lumière.
Après lavage, chaque culot a été resuspendu dans 200 µL de PBS-BSA, et lu au cytomètre de flux (Beckman Coulter Cytomics FC 500).
L'intensité moyenne de fluorescence (IMF) du contrôle positif a été rapportée en fonction de la concentration en IgG (courbe standard) pour des concentrations allant de 0,23 g/L à 30 g/L. Les résultats sont exprimés comme le rapport entre la pente de l'échantillon et la pente du standard positif. L'équation de la courbe standard est y = ax + b; où "a" est la valeur de la pente de la courbe standard et "b", le point zéro correspondant au bruit de fonds de l'essai. Comme l'équation de l'échantillon est y' = a'x + b, et en utilisant les valeurs connues de IMF de l'échantillon (y') et la concentration en IgG (x'), le rapport des pentes a été calculé comme étant [(IMF-b) / [IgG concentration]] / a.

### Résultats :

Le rendement en IgG et l'activité résiduelle anti-A et anti-B obtenus sont présentés dans le tableau ci-dessous :

**Tableau des résultats :**

| | **Vol (mL)** | **Conc. (g/L) IgG** | **Quantité (g) IgG** | **Charge g/mg ligands gel A+B** | **Cytomètre calcul IgG Anti-A Act.résid** | **Cytomètre calcul IgG Anti-B Act.résid** |
|---|---|---|---|---|---|---|
| Départ | **180,0** | **11,60** | **2,1** | **6,8** | **5,14** | **3,44** |
| FNA | **183,8** | **11,10** | **2,0** | | **0,60** | **0,29** |
| Lavage + E + R | **18,8** | **1,20** | **0,0** | | **12%** | **8%** |

Les résultats de ce tableau montrent un rendement en IgG de 98% dans la fraction non adsorbée, ce qui prouve que le support d'affinité présente une bonne spécificité vis-à-vis des isoagglutinines anti-A et anti-B.
Les résultats de ce tableau montrent également une réduction importante de l'activité résiduelle anti-A et anti-B, qui sont respectivement de 12% et 8%. Le produit ainsi obtenu est alors conforme (à la dilution 1/64) à un résultat négatif au test de Coombs direct mis en œuvre avec une concentration initiale d'IgG à 30g/l.

## Revendications

1. Matrice de chromatographie d'affinité, sous forme de gel, comprenant des particules de polymère sur lesquelles au moins un oligosaccharide correspondant à un épitope de groupe sanguin A et/ou de groupe B est greffé, ledit oligosaccharide étant greffé auxdites particules via un espaceur, **caractérisée en ce que** la densité d'oligosaccharides est d'environ 0,3 à 0,4 mg/ml de matrice et ledit espaceur consiste en un espaceur de formule -NH-C₅H₁₀-CO-NH-C₃H₆-, ledit espaceur étant lié par sa fonction amine à la particule..

2. Matrice selon la revendication 1, comprenant (i) des particules de polymère sur lesquelles au moins un oligosaccharide correspondant à un épitope de groupe sanguin A est greffé, et/ou (ii) des particules de polymère sur lesquelles au moins un oligosaccharide correspondant à un épitope de groupe sanguin B est greffé.

3. Matrice selon la revendication 2, comprenant (i) des particules de polymère sur lesquelles au moins un oligosaccharide correspondant à un épitope de groupe sanguin A est greffé, et (ii) des particules de polymère sur lesquelles au moins un oligosaccharide correspondant à un épitope de groupe sanguin B est greffé.

4. Matrice selon la revendication 1, comprenant des particules de polymère sur lesquelles à la fois au moins un oligosaccharide correspondant à un épitope de groupe sanguin A, et au moins un oligosaccharide correspondant à un épitope de groupe sanguin B, sont greffés.

5. Matrice selon la revendication 1, comprenant un mélange de (i) particules de polymère sur lesquelles au moins un oligosaccharide correspondant à un épitope de groupe sanguin A est greffé, (ii) particules de polymère sur lesquelles au moins un oligosaccharide correspondant à un épitope de groupe sanguin B est greffé, et (iii) particules de polymère sur lesquelles à la fois au moins un oligosaccharide correspondant à un épitope de groupe sanguin A, et au moins un oligosaccharide correspondant à un épitope de groupe sanguin B, sont greffés.

6. Matrice selon l'une des revendications 1 à 5, dans laquelle la densité d'oligosaccharides est d'environ 0,3mg/ml de matrice.

7. Matrice selon l'une des revendications 1 à 6, dans laquelle l'oligosaccharide correspondant à un épitope de groupe sanguin A et/ou l'oligosaccharide correspondant à un épitope de groupe sanguin B est un trisaccharide.

8. Matrice selon la revendication 7, dans laquelle l'oligosaccharide correspondant à un épitope de groupe sanguin A est un trisaccharide N-acétylgalactosamine (GalNAc)-Galactose (Gal)-Fucose.

9. Matrice selon la revendication 7, dans laquelle l'oligosaccharide correspondant à un épitope de groupe sanguin B est un oligosaccharide Galactose-Galactose-Fucose.

10. Matrice selon la revendication 3, dans laquelle les particules de polymère sur lesquelles au moins un oligosaccharide correspondant à un épitope de groupe sanguin A est greffé, et les particules de polymère sur lesquelles au moins un oligosaccharide correspondant à un épitope de groupe sanguin B est greffé, sont mélangées dans une proportion de 25/75 à 75/25 (v/v), de préférence environ 50/50 (v/v).

11. Matrice selon l'une des revendications 1 à 10, dans laquelle le polymère est un polymère réticulé, de préférence de la cellulose, et les particules sont de préférence des billes de cellulose poreuses.

12. Utilisation de la matrice selon l'une des revendications 1 à 11, dans une chromatographie d'affinité liant des anticorps anti-A et/ou anti-B, de préférence pour la production à l'échelle industrielle d'immunoglobulines G (IgG) à usage thérapeutique.

13. Procédé de préparation d'un concentré d'immunoglobulines G (IgG) à usage thérapeutique, comprenant l'obtention d'une composition d'Ig à partir de plasma sanguin, par fractionnement éthanolique et/ou fractionnement caprylique et/ou séparation chromatographique, et l'élimination des anticorps anti- A et/ou anti- B éventuellement présents dans la composition, au moyen d'une chromatographie d'affinité mettant en œuvre la matrice selon l'une des revendications 1 à 11.

## Patentansprüche

1. Affinitätschromatographiematrix in Form eines Gels, umfassend Polymerpartikel, auf die wenigstens ein Oligosaccharid gepfropft ist, das einem Epitop der Blutgruppe A und/oder der Blutgruppe B entspricht, wobei besagtes Oligosaccharid auf die Partikel über einen Spacer gepfropft ist, **dadurch gekennzeichnet, dass** die Dichte der Oligosaccharide ungefähr 0,3 bis 0,4 mg/ml Matrix beträgt und besagter Spacer aus einem Spacer der Formel -NH-C₅H₁₀-CO-NH-C3H6- besteht, wobei besagter Spacer über seine Aminfunktion an das Partikel gebunden ist.

2. Matrix gemäß Anspruch 1, umfassend (i) Polymerpartikel, auf die wenigstens ein Oligosaccharid gepfropft ist, das einem Epitop der Blutgruppe A entspricht, und/oder (ii) Polymerpartikel, auf die wenigstens ein Oligosaccharid gepfropft ist, das einem Epitop der Blutgruppe B entspricht.

3. Matrix gemäß Anspruch 2, umfassend (i) Polymerpartikel, auf die wenigstens ein Oligosaccharid gepfropft ist, das einem Epitop der Blutgruppe A entspricht, und (ii) Polymerpartikel, auf die wenigstens ein Oligosaccharid gepfropft ist, das einem Epitop der Blutgruppe B entspricht.

4. Matrix gemäß Anspruch 1, umfassend Polymerpartikel, auf die zugleich wenigstens ein Oligosaccharid, das einem Epitop der Blutgruppe A entspricht, und wenigstens ein Oligosaccharid, das einem Epitop der Blutgruppe B entspricht, gepfropft sind.

5. Matrix gemäß Anspruch 1, umfassend ein Gemisch von (i) Polymerpartikeln, auf die wenigstens ein Oligosaccharid gepfropft ist, das einem Epitop der Blutgruppe A entspricht, (ii) Polymerpartikeln, auf die wenigstens ein Oligosaccharid gepfropft ist, das einem Epitop der Blutgruppe B entspricht, und (iii) Polymerpartikeln, auf die zugleich wenigstens ein Oligosaccharid, das einem Epitop der Blutgruppe A entspricht, und wenigstens ein Oligosaccharid, das einem Epitop der Blutgruppe B entspricht, gepfropft sind.

6. Matrix gemäß einem der Ansprüche 1 bis 5, wobei die Dichte der Oligosaccharide ungefähr 0,3 mg/ml Matrix beträgt.

7. Matrix gemäß einem der Ansprüche 1 bis 6, wobei das Oligosaccharid, das einem Epitop der Blutgruppe A entspricht, und/oder das Oligosaccharid, das einem Epitop der Blutgruppe B entspricht, ein Trisaccharid ist.

8. Matrix gemäß Anspruch 7, wobei das Oligosaccharid, das einem Epitop der Blutgruppe A entspricht, ein N-Acetylgalactosamin (GalNAc)-Galactose (Gal)-Fucose-Trisaccharid ist.

9. Matrix gemäß Anspruch 7, wobei das Oligosaccharid, das einem Epitop der Blutgruppe B entspricht, ein Galactose-Galactose-Fucose-Oligosaccharid ist.

10. Matrix gemäß Anspruch 3, wobei die Polymerpartikel, auf die wenigstens ein Oligosaccharid gepfropft ist, das einem Epitop der Blutgruppe A entspricht, und die Polymerpartikel, auf die wenigstens ein Oligosaccharid gepfropft ist, das einem Epitop der Blutgruppe B entspricht, in einem Verhältnis von 25/75 bis 75/25 (v/v), vorzugsweise ungefähr 50/50 (v/v) gemischt sind.

11. Matrix gemäß einem der Ansprüche 1 bis 10, wobei das Polymer ein vernetztes Polymer ist, vorzugsweise Cellulose, und die Partikel vorzugsweise poröse Cellulosekügelchen sind.

12. Verwendung der Matrix gemäß einem der Ansprüche 1 bis 11, in einer Affinitätschromatographie, die anti-A- und/oder anti-B-Antikörper bindet, vorzugsweise zur großtechnischen Herstellung von Immunglobulinen G (IgG) zur therapeutischen Verwendung.

13. Verfahren zur Herstellung eines Konzentrats von Immunglobulinen G (IgG) zur therapeutischen Verwendung, umfassend den Erhalt einer Ig-Zusammensetzung aus Blutplasma mittels Ethanol-Fraktionierung und/oder Caprylsäure-Fraktionierung und/oder chromatographischer Trennung und die Elimination der anti-A- und/oder anti-B-Antikörper, die möglicherweise in der Zusammensetzung vorhanden sind, mithilfe einer Affinitätschromatographie, die die Matrix gemäß einem der Ansprüche 1 bis 11 verwendet.

## Claims

1. An affinity chromatography matrix, in the form of a gel, comprising polymer particles on which at least one oligosaccharide corresponding to a blood group A and/or group B epitope is grafted, said oligosaccharide being grafted to said particles via a spacer, **characterized in that** the oligosaccharide density is about 0.3 to 0.4 mg/ml matrix and said spacer consists of a spacer of the formula -NH-C₅H₁₀-CO-NH-C₃H₆-, said spacer being bound through its amine function to the particle.

2. The matrix of claim 1, comprising (i) polymer particles on which at least one oligosaccharide corresponding to a blood group A epitope is grafted, and/or (ii) polymer particles on which at least one oligosaccharide corresponding to a blood group B epitope is grafted.

3. The matrix of claim 2, comprising (i) polymer particles on which at least one oligosaccharide corresponding to a blood group A epitope is grafted, and (ii) polymer particles on which at least one oligosaccharide corresponding to a blood group B epitope is grafted.

4. The matrix of claim 1, comprising polymer particles on which both at least one oligosaccharide corresponding to a blood group A epitope and at least one oligosaccharide corresponding to a blood group B epitope are grafted.

5. The matrix of claim 1, comprising a mixture of (i) polymer particles on which at least one oligosaccharide corresponding to a blood group A epitope is grafted, (ii) polymer particles on which at least one oligosaccharide corresponding to a blood group B epitope is grafted, and (iii) polymer particles on which both at least one oligosaccharide corresponding to a blood group A epitope, and at least one oligosaccharide corresponding to a blood group B epitope, are grafted.

6. The matrix of one of claims 1 to 5, wherein the oligosaccharide density is about 0.3 mg/ml matrix.

7. The matrix of one of claims 1 to 6, wherein the oligosaccharide corresponding to a blood group A epitope and/or the oligosaccharide corresponding to a blood group B epitope is a trisaccharide.

8. The matrix of claim 7, wherein the oligosaccharide corresponding to a blood group A epitope is a N-acetylgalactosamine (GalNAc)-Galactose (Gal)-Fucose trisaccharide.

9. The matrix of claim 7, wherein the oligosaccharide corresponding to a blood group B epitope is a Galactose-Galactose-Fucose oligosaccharide.

10. The matrix of claim 3, wherein the polymer particles on which at least one oligosaccharide corresponding to a blood group A epitope is grafted, and the polymer particles on which at least one oligosaccharide corresponding to a blood group B epitope is grafted, are mixed in a proportion of 25/75 to 75/25 (v/v), preferably about 50/50 (v/v).

11. The matrix of one of claims 1 to 10, wherein the polymer is a cross-linked polymer, preferably cellulose, and the particles are preferably porous cellulose beads.

12. Use of the matrix of one of claims 1 to 11, in affinity chromatography binding anti-A and/or anti-B antibodies, preferably for the industrial-scale production of immunoglobulin G (IgG) for therapeutic use.

13. A process for preparing an immunoglobulin G (IgG) concentrate for therapeutic use, comprising obtaining an Ig composition from blood plasma, by ethanolic fractionation and/or caprylic fractionation and/or chromatographic separation, and removing anti-A and/or anti-B antibodies possibly present in the composition, by means of affinity chromatography using the matrix of one of claims 1 to 11.
